(19) Europäisches Patentamt
European Patent Office
Office européen des brevets

(11) Veröffentlichungsnummer: **0 025 869**
**B1**

# (12) EUROPÄISCHE PATENTSCHRIFT

(45) Veröffentlichungstag der Patentschrift: **28.09.83**

(51) Int. Cl.³: **C 07 C 49/587**, C 07 C 45/65, A 61 K 7/46

(21) Anmeldenummer: **80104889.3**

(22) Anmeldetag: **16.08.80**

## (54) 3-Methyl-cyclohexadecen-5-on-1, Verfahren zu seiner Herstellung und seine Verwendung als Riechstoff.

(30) Priorität: **28.08.79 DE 2934683**

(43) Veröffentlichungstag der Anmeldung: **01.04.81 Patentblatt 81/13**

(45) Bekanntmachung des Hinweises auf die Patenterteilung: **28.09.83 Patentblatt 83/39**

(84) Benannte Vertragsstaaten: **CH DE FR GB IT LI NL**

(56) Entgegenhaltungen:
**DE-A-2 165 113**
**DE-B-1 793 801**
**DE-B-2 141 309**

(73) Patentinhaber: **HAARMANN & REIMER GMBH, Postfach 138, D-3450 Holzminden (DE)**

(72) Erfinder: **Bauer, Kurt, Dr., Corveyblick 41, D-3450 Holzminden (DE)**
Erfinder: **Körber, Alfred, Dr., Bismarckstrasse 4, D-3450 Holzminden (DE)**
Erfinder: **Oelkers, Egon, Rotdornstrasse 15, D-3454 Bevern (DE)**
Erfinder: **Bork, Karl-Heinz, Kurze Breite 10, D-3457 Deensen (DE)**

(74) Vertreter: **Dill, Erwin, Dr. et al, c/o BAYER AG Zentralbereich Patente Marken und Lizenzen Bayerwerk, D-5090 Leverkusen 1 (DE)**

EP 0 025 869 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

3-Methyl-cyclohexadecen-5-on-1, Verfahren zu seiner Herstellung und seine Verwendung als Riechstoff

Die Erfindung betrifft 3-Methyl-cyclohexadecen-5-on-1, ferner ein Verfahren zu seiner Herstellung. Das Verfahren ist dadurch gekennzeichnet, daß man eine Verbindung der allgemeinen Formel

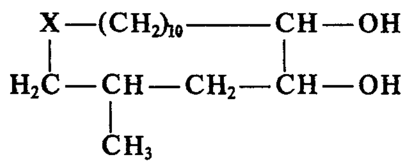

(I)

in der

X eine Keto- oder eine Ketalgruppe darstellt, mit einer Lösung von Bromwasserstoffsäure in Eisessig in die Bromacetoxiverbindung und diese durch Behandeln mit Zinkstaub in 3-Methyl-cyclohexadecen-5-on-1 überführt.

Außerdem betrifft die Erfindung die Verwendung von 3-Methyl-cyclohexadecen-5-on-1 als Riechstoff.

Als Verbindungen der allgemeinen Formel I kommen 4-Methyl-6-oxo-cyclohexadecan-1,2-diol und die entsprechenden Ketale, wie z. B. das Dimethyl-, Diethyl-, vorzugsweise das Ethylenketal in Frage. Die Umsetzung der Oxoverbindung zu den Ketalen erfolgt unter den aus Tetrahedron 20, 2601 (1964) bekannten Bedingungen.

Die Herstellung der Verbindungen der Formel (I) kann nach folgendem Verfahren erfolgen:

Dodecandisäure wird nach bekannten Verfahren in das Halbesterchlorid überführt. Das Halbesterchlorid wird mit Dimethylacrylsäureethylester in Gegenwart eines Friedel-Crafts-Katalysators zu einem Isomerengemisch aus 3-Methyl-5-oxo-hexadecen-2-disäurediethylester und 3-Methyl-5-oxo-hexadecen-3-disäureethylester umgesetzt. Dieses Estergemisch wird durch Hydrieren in Gegenwart von Raney-Nickel und anschließende Maskierung der Oxogruppe durch Ketalbildung mit einem niederen ein- oder zweiwertigen Alkohol in Gegenwart von p-Toluolsulfonsäure in 3-Methyl-5-dialkoxi- bzw. 3-Methyl-5-alkylendioxi-hexadecandisäureethylester überführt. Durch Acyloinkondensation dieses Hexadecandisäurediesters und nachfolgende Reduktion des Acyloins mit Lithiumaluminiumhydrid werden die Verbindungen der Formel I erhalten, in der X für eine Ketalgruppe steht. Diese Ketale lassen sich durch saure Hydrolyse in das entsprechende Keton, d. h. die Verbindung der Formel I, in der X für eine Ketogruppe steht, überführen. Sowohl Keton wie Ketale sind in dem erfindungsgemäßen Herstellungsverfahren einsetzbar.

Das erfindungsgemäße 3-Methyl-cyclohexadecen-5-on-1 ist ein wertvoller Riechstoff, der eine sehr natürliche Moschusnote aufweist, die an Moschustinktur, einen alkokolischen Auszug aus Moschusbeuteln, erinnert. Er ist daher besonders geeignet, als Komponente zum Aufbau eines Moschustinkturersatzes eingesetzt zu werden. Darüber hinaus ist er wesentlich haftfester als das für diesen Zweck bisher verwendete Muscon.

Aus der DE-B-2 141 309 und der DE-A-2 165 113 ist das Cyclohexadecen-5-on-1 als Riechstoff bekannt. Es weist eine typisch makrocyclische (synthetische) Moschusnote auf. Dagegen hat das erfindungsgemäße 3-Methyl-cyclohexadecen-5-on-1 überraschenderweise eine natürliche Moschusnote und eine bessere Haftfestigkeit.

Die erfindungsgemäße Verbindung wird in Mischungen mit anderen Riechstoffen in Riechstoffkompositionen, z. B. in Mengen von 0,01 bis 25 Gewichtsprozent, bezogen auf das Gesamtgewicht eingesetzt.

Das Anwendungsgebiet der erfindungsgemäßen Verbindung ist wegen ihres harmonischen Geruchstyps und ihrer vorteilhaften anwendungstechnischen Eigenschaften, z. B. der Haftfestigkeit und der Stabilität gegenüber aggressiven Medien, ungewöhnlich groß. Sie eignet sich für Verwendung in Parfümkompositionen für die verschiedensten Fertigungsprodukte, z. B. für hochwertige Kosmetika, für Feinparfümerieartikel wie Extraits, Seifen, Deo-Sprays, Shampoos, Schaumbäder und für Detergentien.

## Beispiel 1

76 g (0,23 Mol) 4-Methyl-6-ethylendioxi-cyclohexadecan-1,2-diol werden mit 400 ml einer 30%igen Lösung von Bromwasserstoffsäure in Eisessig 24 Stunden bei Raumtemperatur gerührt und dann 3 Stunden auf 65°C erwärmt. Nach Zugabe von 80 g Essigsäureanhydrid wird die Mischung weitere 3 Stunden auf 65°C erwärmt. Nach dem Abkühlen wird die überschüssige Bromwasserstoffsäure mit 180 g Natriumacetat in 1,3 l Wasser neutralisiert. Anschließend wird die Reaktionsmischung mit Petrolether extrahiert. Nach dem Abdestillieren des Petrolethers erhält man 83 g einer Mischung von 2-Acetoxi-4-methyl-6-oxo-cyclohexadecan-1-bromid und 16-Acetoxi-3-methyl-5-oxo-cyclohexadecan-1-bromid.

Diese Mischung wird ohne weitere Reinigung in 650 ml absolutem Methanol gelöst und nach Zugabe von 60 g Zinkstaub unter Rühren 12 Stunden auf Rückflußtemperatur erhitzt werden. Nach dem Abdestillieren des Methanols wird der Rückstand in Petrolether aufgenommen und filtriert. Das Filtrat wird zunächst mit verdünnter Essigsäure, danach mit Wasser säurefrei gewaschen. Der nach Abziehen des Lösungsmittels verbleibende Rückstand wird fraktioniert destilliert. Man erhält 65 g eines cis-trans-Gemisches von 3-Methyl-cyclohexadecen-5-on-1 mit einem Siedepunkt von 155–157° C/2 m bar.

Das als Ausgangsmaterial verwendete 4-Methyl-6-ethylendioxi-cyclohexadecan-1,2-diol wird nach folgendem Verfahren hergestellt:

Eine Mischung von 276,5 g (1 Mol) $\alpha,\omega$-Dodecandisäuremonoethylesterchlorid und 128 g (1 Mol) $\beta,\beta$-Dimethylacrylsäureethylester wird bei 30 bis 35° C im Verlauf von 1,5 Stunden zu einer Suspension von 399 g (2,99 Mol) Aluminiumchlorid in 300 ml Methylenchlorid getropft. Nach beendeter Zugabe wird die Reaktionsmischung 3 Stunden auf 45 bis 50° C erwärmt. Dann wird das Reaktionsgemisch mit Eis hydrolysiert und das Reaktionsprodukt mit Methylenchlorid extrahiert. Nach Abdestillieren des Lösungsmittels werden 370,4 g Rohprodukt erhalten.

Nach mehrmaligem Umkristallisieren des Rohproduktes aus Methanol werden 191,1 g 3-Methyl-5-oxo-hexadecen-2-disäurediethylester (Fp.: 58–59° C) erhalten.

191,1 g (0,52 Mol) 3-Methyl-5-oxo-hexadecen-2-disäurediethylester werden in 1250 ml Methanol gelöst und nach Zusatz von 20 g Raney-Nickel bei 20° C und unter einem Wasserstoffdruck von 40,5 bar hydriert. Nach dem Abfiltrieren des Katalysators wird das Methanol destilliert. Es werden 186 g 3-Methyl-5-oxo-hexadecandisäurediethylester (Fp.: 25° C) erhalten.

Die 186 g 3-Methyl-5-oxo-hexadecandisäurediethylester werden mit 230 ml Ethylenglykol und 1 g p-Toluolsulfonsäure in 6,3 l Benzol am Wasserabscheider auf Rückflußtemperatur erhitzt. Nach 16 Stunden scheidet sich kein Wasser mehr ab. Die benzolische Lösung wird neutralgewaschen und vom überschüssigem Ethylenglykol befreit. Nach Abziehen des Lösungsmittels verbleiben 219 g Rohprodukt, die gemäß Gaschromatogramm zu 84% aus 3-Methyl-5-ethylendioxi-hexadecandisäurediethylester (Kp.: 219° C/0,94 mbar) bestehen.

201,2 g (0,486 Mol) dieses Esters in 200 ml Xylol werden innerhalb von 5 Stunden bei Rückflußtemperatur zu einer Suspension von 40,89 g Natrium in 3,6 l Xylol getropft.

Nach Abkühlen werden 108 g Eisessig in 110 ml Xylol zugetropft. Das Reaktionsgemisch wird mit Wasser neutral gewaschen und das Lösungsmittel abgezogen. Die Destillation des Rückstandes liefert 123,5 g eines Isomerengemisches von 2-Hydroxy-4-methyl-6-ethylendioxi-cyclohexadecan-on-1 und 16-Hydroxy-3-methyl-5-ethylendioxi-cyclohexadecan-on-1 mit einem Siedepunkt von 185–190° C/0,5 mbar.

78 g dieses Isomerengemisches, gelöst in 200 ml Ether, werden innerhalb 30 Minuten zu einer Suspension von 20 g Lithiumaluminiumhydrid in 100 ml Ether unter Eiskühlung getropft. Dann wird 10 Stunden auf Rückflußtemperatur erhitzt. Anschließend wird überschüssiges Lithiumaluminiumhydrid mit feuchtem Ether zersetzt und das Reaktionsgemisch mit Wasser neutralgewaschen.

Nach Abziehen des Lösungsmittels erhält man 78 g 4-Methyl-6-ethylendioxy-cyclohexadecan-1,2-diol als viskoses Öl, das auch im Hochvakuum nicht ohne Zersetzung destillierbar ist.

## Beispiel 2

Eine blumige Phantasiekomposition wird durch Mischen folgender Komponenten hergestellt:

5 Evernyl (Handelsprodukt der Fa. Roure-Bertrand)
3 2,6,10-Trimethyl-9-undecen-1-al
5 Undecalacton, 10%ig in Diethylphthalat
60 Linalylacetat
35 Geranylacetat
5 Phenylacetaldehyd, 50%ig in Diethylphthalat
5 Rosenoxid, 10%ig in Diethylphthalat
30 1-Citronellol
20 Geraniol
5 Rosenöl
20 Geraniumöl afrikanisch
50 Phenylethylalkohol
10 Phenylessigsäuregeranylester
30 Benzylacetat
150 Methyldihydrojasmonat
2 Jasmin absolue marokkanisch
100 4-(4-Hydroxi-4-methyl-pentyl)-3-cyclohexencarboxaldehyd
10 $\alpha$-Methyl-3,4-methylendioxi-hydrozimtaldehyd
50 $\gamma$-Iraldein

10 Styraxöl
30 Benzylsalicylat
120 Vetiverylacetat
50 Sandelholzöl ostindisch
20 Moschus Keton
15 Cumarin
840 Gewichtsteile

Durch Zugabe von 30 Gewichtsteilen 3-Methylcyclohexadecen-5-on-1 erhält die Komposition eine erogene, warme, natürliche Moschusnote, die im Nachgeruch besonders zum Tragen kommt und die die feminine Note der Komposition unterstützt.

**Patentansprüche**

1. 3.-Methyl-cyclohexadecen-5-on-1.

2. Verfahren zur Herstellung von 3-Methyl-cyclohexadecen-5-on-1, dadurch gekennzeichnet, daß man eine Verbindung der allgemeinen Formel

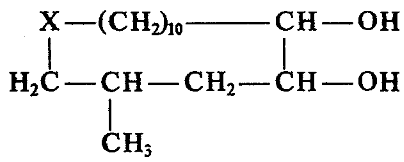

in der
X für eine Keto- oder eine Ketalgruppe steht, mit einer Lösung von Bromwasserstoffsäure in Eisessig in die Bromacetoxiverbindung und diese durch Behandeln mit Zinkstaub in 3-Methyl-cyclohexadecen-5-on-1 überführt.

3. Verwendung von 3-Methyl-cyclohexadecen-5-on-1 als Riechstoff.

**Claims**

1. 3-Methyl-cyclohexadec-5-en-1-one.

2. Process for the preparation of 3-methyl-cyclohexadec-5-en-1-one, characterised in that a compound of the general formula

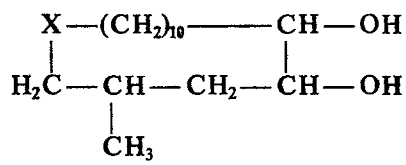

in which
X represents a keto group or a ketal group, is converted into the bromoacetoxy compound with a solution of hydrobromic acid in glacial acetic acid, and this compound is converted into 3-methyl-cyclohexadec-5-en-1-one by treatment with zinc dust.

3. Use of 3-methyl-cyclohexadec-5-en-1-one as an odoriferous substance.

**Revendications**

1. La 3-méthyl-cyclohexadécène-5-one-1.

2. Procédé de production de la 3-méthyl-cyclohexadécène-5-one-1, caractérisé en ce qu'on transforme en le composé bromacétoxy un composé de formule générale

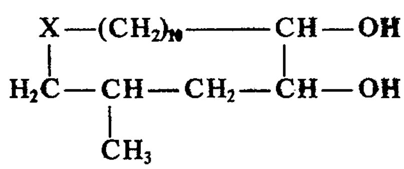

dans laquelle
X représente un groupe céto ou un groupe cétal, avec une solution d'acide bromhydrique dans l'acide acétique cristallisable et on transforme ce composé en 3-méthyl-cyclohexadécène-5-one-1 par traitement avec du zinc en poudre.

3. Utilisation de la 3-méthyl-cyclohexadécène-5-one-1-comme parfum.